# EUROPEAN PATENT APPLICATION

(11) **EP 1 915 995 A1**
(43) Date of publication of application: **30.04.2008**
(21) Application number: 06781644.7
(22) Date of filing: 26.07.2006
(51) Int. Cl.: A61K 31/4745, A61K 9/08, A61K 47/02, A61P 35/00

(54) **AQUEOUS SOLUTION PREPARATION CONTAINING CAMPTOTHECINS**

(30) Priority: 27.07.2005 JP 2005217259
(71) Applicant: KABUSHIKI KAISHA YAKULT HONSHA, Minato-ku, Tokyo 105-8660 (JP)
(72) Inventor: NAKAZAWA, Masako, Kabushiki Kaisha Yakult Honsha, Tokyo 1058660 (JP); AIYAMA, Ritsuo, Kabushiki Kaisha Yakult Honsha, Tokyo 1058660 (JP)
(74) Representative: Hartz, Nikolai
(86) International application number: PCT/JP2006/314732
(87) International publication number: WO 2007/013490

(57) **Abstract**

An object of the invention is to provide a camptothecin compounds-containing aqueous pharmaceutical preparation which can be produced without heating and in which camptothecin compounds are dissolved in a stable state. The invention provides a camptothecin compound-containing aqueous pharmaceutical preparation, containing the following ingredients (a) to (c):
(a) a camptothecin compound;
(b) a phosphoric acid salt; and
(c) phosphoric acid.

## Description

### Technical Field

The present invention relates to a stable pharmaceutical preparation in the form of aqueous solution (hereinafter referred to as "aqueous pharmaceutical preparation") which exhibits high solubility of camptothecin compounds therein.

### Background Art

Camptothecin (CPT) is an alkaloid contained in fruit, roots, etc. of *Camptotheca acuminata*, a tree native to China. Also, 7-ethyl-10-piperidinopiperidinocarbonyloxycamptothecin (CPT-11) (see Patent Document 1), which is a semi-synthesized derivative of camptothecin, is a particularly valuable substance, being a compound which exhibits high anti-tumor activity inherent to camptothecin and yet has reduced toxicity. The activity of 7-ethyl-10-piperidinopiperidinocarbonyloxycamptothecin is considered to be exhibited after 7-ethyl-10-piperidinopiperidinocarbonyloxycamptothecin has been metabolized in the living body to form a semi-synthesized derivative, 7-ethyl-10-hydroxycamptothecin (SN-38) (see Patent Document 2).

Camptothecin compounds including 7-ethyl-10-piperidinopiperidinocarbonyloxycamptothecin are administered to a patient in need thereof primarily via intravenous injection. Therefore, camptothecin compounds (e.g., 7-ethyl-10-piperidinopiperidinocarbonyloxycamptothecin) currently available on the market or distributed for use usually take the form of an isotonic solution prepared in combination with a substance such as sorbitol. A variety of attempts to prepare a drug product containing camptothecin compounds have heretofore been carried out. Examples of the drug product include a sustained-release drug prepared by incorporating a camptothecin derivative into a copolymer of collagen and 2-hydroxyethylmethacrylate (see Patent Document 3), and a sustained-release drug prepared by incorporating camptothecin or a derivative thereof into a carrier formed of a poly(lactic acid-glycolic acid) copolymer (see Patent Document 4).
However, since camptothecin compounds have poor solubility in water, the production of aqueous pharmaceutical preparations thereof requires a heating process. Thus, in order to simplify production steps, there is demand for developing an aqueous pharmaceutical preparation of camptothecin compounds, which preparation can be produced without heating.
Patent Document 1:
   Japanese Patent Publication (kokoku) No. 3-4077
Patent Document 2:
   Japanese Patent Publication (kokoku) No. 62-47193
Patent Document 3:
   Japanese Patent Publication (kokoku) No. 7-277981
Patent Document 4:
   Japanese Patent Publication (kokoku) No. 10-17472

### Disclosure of the Invention

### Problems to be Solved by the Invention

An object of the present invention is to provide an aqueous pharmaceutical preparation containing camptothecin compounds which can be produced without heating and in which camptothecin compounds are dissolved in a stable state.

### Means for Solving the Problems

The present inventors have conducted extensive studies in order to attain the aforementioned object, and have found that addition of phosphoric acid and a phosphoric acid salt to camptothecin compounds increases the solubility of camptothecin compounds in an aqueous solution, thereby enabling production of a stable camptothecin compounds-containing aqueous pharmaceutical preparation in which camptothecin compounds are dissolved at a concentration higher than that conventionally attained. The present invention has been accomplished on the basis of this finding.
Accordingly, the present invention provides a camptothecin compound-containing aqueous pharmaceutical preparation, containing the following ingredients (a) to (c):
(a) a camptothecin compound;
(b) a phosphoric acid salt; and
(c) phosphoric acid.

### Effects of the Invention

The aqueous pharmaceutical preparation of the present invention is able to dissolve camptothecin compounds at high concentrations without heating during the production thereof. Best Modes for Carrying Out the Invention

The camptothecin compound (a) employed in the present invention is an active ingredient of the aqueous pharmaceutical preparation of the invention. Examples of the camptothecin compound (a) include those naturally occurring such as 10-hydroxycamptothecin and 11-methoxycamptothecin, and chemically modified compounds of natural camptothecin such as 7-ethyl-10-piperidinopiperidinocarbonyloxycamptothecin (hereinafter may be referred to as "CPT-11"). Among them, camptothecin derivatives having an E-ring-closed structure and exhibiting solubility in an acidic medium are preferred as ingredient (a). Of these, CPT-11 is more preferred.

The aqueous pharmaceutical preparation of the present invention is required to contain a phosphoric acid salt, ingredient (b), and phosphoric acid, ingredient (c), in combination.
Examples of the phosphoric acid salt serving as ingredient (b) include those of an alkali metal such as sodium, potassium, or lithium; and those of an alkaline earth metal such as magnesium or calcium. Of these, alkali metal salts are preferred, with sodium salts and potassium salts being more preferred.

The phosphoric acid salt may be formed by adding an alkali agent to phosphoric acid-contained in an aqueous pharmaceutical preparation. Examples of the alkali agent include potassium hydroxide, sodium hydroxide, calcium hydroxide, sodium carbonate, and sodium hydrogencarbonate, with sodium hydroxide being preferred. Alternatively, a phosphoric acid salt may be formed in an aqueous pharmaceutical preparation through salt exchange with another compound.

In the aqueous pharmaceutical preparation of the present invention, the total amount of phosphoric acid salt (b) and phosphoric acid (c), as reduced to phosphoric acid, is preferably 0.1 to 10 wt.%, more preferably 0.5 to 8 wt.%, more preferably 0.7 to 6 wt.%.
The aqueous pharmaceutical preparation of the present invention preferably contains the phosphoric acid salt (b) and phosphoric acid (c) in a total amount, as reduced to phosphoric acid and with respect to 100 mg camptothecin compounds, of 50 to 1,000 mg, more preferably 200 to 500 mg, so as to enhance the solubility of camptothecin compounds in the aqueous pharmaceutical preparation.

The aqueous pharmaceutical preparation of the present invention may further contain one, two or more species selected from among propylene glycol, dimethylacetamide, dimethylformamide, ethanol, sodium hydrogensulfite, sodium thioglycolate, potassium pyrosulfite, sodium pyrosulfite, α-thioglycerin, ethylene glycol, dimethylsulfoxide, sodium sulfite, and sodium erythorbate, whereby the solubility and stability of camptothecin compounds in the aqueous can be remarkably enhanced. Among them, incorporation of propylene glycol or dimethylacetamide is particularly preferred.

No particular limitation is imposed on the amount of the above additive(s) incorporated into the aqueous pharmaceutical preparation, and the amount is preferably 0.1 to 80 wt.%, more preferably 0.3 to 70 wt.%, even more preferably 0.5 to 60 wt.%.
When dimethylacetamide is used, the amount thereof is preferably 3 to 15 wt.%, more preferably 5 to 10 wt.%, whereas when propylene glycol is used, the amount thereof is preferably 5 to 60 wt.%, more preferably 10 to 20 wt.%.

The aqueous pharmaceutical preparation of the present invention preferably contains the above compound(s) in an amount, with respect to 100 mg camptothecin compounds, of 0.005 to 4 g, more preferably 0.015 to 3.5 g, even more preferably 0.025 to 3 g, so as to enhance the solubility of camptothecin compounds in the aqueous pharmaceutical preparation. When propylene glycol is employed, the amount thereof is preferably 0.25 g to 3 g, more preferably 0.5 to 2 g.

The aqueous pharmaceutical preparation of the present invention preferably has a pH of 2 to 5 at room temperature (25°C), more preferably 2.5 to 4.8, so as to attain desired solubility of camptothecin compounds. The pH is preferably adjusted by use of an acid such as phosphoric acid, hydrochloric acid, sulfuric acid, acetic acid, lactic acid, or malic acid, or a sodium-containing alkali such as sodium hydroxide, sodium carbonate, or sodium hydrogencarbonate.

The aqueous pharmaceutical preparation of the present invention is useful as an anti-tumor drug, because its active ingredients, camptothecin compounds, have an excellent malignant tumor therapeutic effect. Examples of target malignant tumors include lung cancer, uterus cancer, ovarian cancer, gastric cancer, colonic/rectum cancer, breast cancer, lymphoma, and pancreatic cancer.

Preferably, the aqueous pharmaceutical preparation of the present invention takes a product form of injection liquid, more preferably that for intravenous injection. When an injection product is prepared, in addition to the above-described ingredients, there may be incorporated other additives including distilled water for injection; sugars such as glucose, mannose, and lactose; inorganic salts such as common salt; organic amines such as HEPES and PIPES; and other ingredients which are generally employed in preparation of injections such as a stabilizer, an excipient, and a buffer. In an injection product, camptothecin compounds are preferably contained in 1 to 50 mg/mL, more preferably 10 to 30 mg/mL.

### Examples

The present invention will next be described in more detail by way of examples, which should not be construed as limiting the invention thereto.

### Example 1

To each (10 mL) of the aqueous solutions listed in Table 1, CPT-11 was added in an amount of 250 to 500 mg, and the mixture was ultrasonicated for 10 minutes, whereby CPT-11 was dispersed in water. After dissolution, the liquid was stirred at room temperature for a predetermined period (day(s)). Subsequently, an aliquot of the solution was sampled and centrifuged at 3,000 r/min for 30 minutes. The supernatant was filtered by means of a 0.45-µ filter. An aliquot (1 mL) of the filtrate was correctly measured, and diluted with 90% aqueous methanol to thereby adjust the total volume to 50 mL. The amount of CPT-11 dissolved was determined through HPLC under the following conditions.

HPLC conditions
Column: Symmetry Shield RP18 (3.5 µm, 4.6 × 50 mm)
Column temperature: 50°C
Flow rate: 2.0 mL/min
Mobile phase:
50-mmol/L formate buffer (pH 5.5)/acetonitrile/methanol =
850/100/50 (liquid A);
50-mmol/L formate buffer (pH 5.5)/acetonitrile/methanol =
700/250/50 (liquid B);
Linear gradient of liquid B (0 to 100%) over 15 minutes; and equilibrated with liquid A (100%) over 5 minutes.
Amount of injection: 10 µL
Detection wavelength: 254 nm

After stirring of each aqueous solution at room temperature for 1 day or 2 or 3 days, the amount of CPT-11 dissolved in the solution was determined. Table 1 shows the results. In Table 1, the amount of CPT-11 dissolved in 1 mL of the aqueous solution (CPT-11 mg/mL) is represented by the unit mg/mL.

**[Table 1]**

| No. | Amount (mg) in aq. solution (5 mL) | | | | pH | Day(s) of stirring | |
|---|---|---|---|---|---|---|---|
| | Monosodium phosphate | Phosphoric acid | Dimethylacetamide | Propylene glycol | | 1 | 2 or 3 |
| 1 | 200 | ** | - | - | 2.5 | 19.32 | 19.83* |
| 2. | 200 | ** | - | 1,000 | 2.5 | 26.06 | 21.31* |
| 3 | 200 | ** | 300 | - | 2.5 | 23.75 | 23.73* |
| 4 | 200 | ** | 300 | 1,000 | 2.5 | 28.49 | 23.26* |
| 5 | 200 | ** | 300 | - | 3.0 | 20.55 | 21.42 |
| 6 | 200 | ** | 300 | 1,000 | 3.0 | 28.95 | 22.26* |
| 7 | 200 | ** | - | 1,000 | 3.5 | 19.82 | 20.19 |
| 8 | 200 | ** | 300 | - | 3.5 | 19.74 | 20.92 |
| 9 | 200 | ** | 300 | 1,000 | 3.5 | 26.65 | 21.77* |
| 10 | 200 | ** | 300 | - | 4.0 | 19.91 | 20.18 |
| 11 | 200 | ** | 300 | 1,000 | 4.0 | 27.77 | 22.85* |
| Control | 0 | 0 | - | - | 4.0 | 14.04 | 13.83 |

| | | | | | | | |
|---|---|---|---|---|---|---|---|
| Note: *: Sample stirred for 3 days **: Amount required for adjusting pH | | | | | | | |

All the camptothecin compound-containing aqueous pharmaceutical preparations of the present invention (Nos. 1 to 11) exhibited excellent CPT-11 solubility. When these aqueous pharmaceutical preparations were maintained at room temperature (25°C) for 3 days without any light shielding, no coloration was observed, and no crystals were deposited.
When vibration was applied to the aqueous liquid pharmaceutical preparations, no deposition of CPT-11 crystals occurred.

### Example 2

To each (10 mL) of the aqueous solutions listed in Table 2, CPT-11 was added in an amount of 250 to 500 mg, and the mixture was unltasonicated for 10 minutes, whereby CPT-11 was dispersed in water. After dissolution, the amount of CPT-11 dissolved in 1 mL of the aqueous solution (CPT-11 mg/mL) was determined in a manner similar to.that of Example 1. Table 2 shows the results.
All the camptothecin-containing aqueous pharmaceutical preparations of the present invention (Nos. 12 to 22) exhibited excellent CPT-11 solubility. When these aqueous pharmaceutical preparations were maintained at room temperature (25°C) for 3 days without any light shielding, no coloration was observed, and no crystals were deposited.
When vibration was applied to the aqueous liquid preparations, no deposition of CPT-11 crystals occurred.

**[Table 2]**

| No. | Amount (mg) in aq. solution (5 mL) | | | | pH | Day(s) of stirring | |
|---|---|---|---|---|---|---|---|
| | Monosodium phosphate | Phosphoric acid | Additive | | | 1 | 2 |
| 12 | 200 | 70 µL | Sodium hydrogensulfite | 200 | 2.2 | 19.25 | 19.99 |
| 13 | 200 | 70 µL | Sodium thioglycolate | 50 | 2.5 | 19.15 | 19.95 |
| 14 | 200 | 70 µL | Potassium pyrosulfite | 200 | 2.4 | 20.17 | 20.41 |
| 15 | 200 | 70 µL | Sodium pyrosulfite | 50 | 2.2 | 18.37 | 19.11 |
| 16 | 200 | 70 µL | α-Thioglycerin | 50 | 2.1 | 18.57 | 19.07 |
| 17 | 200 | 70 µL | Dimethylformamide | 300 | 2.6 | 20.04 | 20.41 |
| 18 | 200 | 70 µL | Ethanol | 300 | 2.5 | 19.34 | 20.66 |
| 19 | 200 | 70 µL | Ethylene glycol | 300 | 2.5 | 16.93 | 17.52 |
| 20 | 200 | 70 µL | Dimethylsulfoxide | 300 | 2.6 | 17.31 | 17.45 |
| 21 | 200 | 70 µL | Sodium sulfite | 100 | 2.5 | 17.19 | 17.68 |
| 22 | 200 | 70 µL | Sodium erythorbate | 50 | 2.3 | 17.79 | 17.95 |

### Example 3

The following injection products 1 to 3 were produced in the following procedure.
Specifically, irinotecan hydrochloride (CPT-11) (100 mg) was added to a solution (3.5 mL) in which additives had been sufficiently dissolved, and the mixture was stirred well for dissolution. The solution containing the additives was added to the resultant liquid so as to adjust the total volume to 5 mL.

| Product Example 1: | |
|---|---|
| Irinotecan hydrochloride | 100 mg |
| Sodium phosphate | 200 mg |
| Phosphoric acid | 70 mg |
| Dimethylacetamide | 300 mg |
| Water for injection use | to make the volume 5 mL |
| pH 3.0 | |

| Product Example 2: | |
|---|---|
| Irinotecan hydrochloride | 100 mg |
| Sodium phosphate | 200 mg |
| Phosphoric acid | 70 mg |
| Propylene glycol | 1,000 mg |
| Water for injection use | to make the volume 5 mL pH 3.0 |

| Product Example 3: | |
|---|---|
| Irinotecan hydrochloride | 100 mg |
| Sodium phosphate | 200 mg |
| Phosphoric acid | 70 mg |
| Dimethylacetamide | 300 mg |
| Sodium hydrogensulfite | 200 mg |
| Water for injection use | to make the volume 5 mL pH 3.0 |

All the camptothecin compound-containing aqueous pharmaceutical preparations (injection products), Product Examples 1 to 3 assumed pale yellow, transparent aqueous solution, in which no crystals of irinotecan hydrochloride were deposited.

## Claims

1. A camptothecin compound-containing aqueous pharmaceutical preparation, comprising the following ingredients (a) to (c):
(a) a camptothecin compound;
(b) a phosphoric acid salt; and
(c) phosphoric acid.

2. The camptothecin compound-containing aqueous pharmaceutical preparation, further comprising ingredient (d) one or more species selected from among propylene glycol, dimethylacetamide, dimethylformamide, ethanol, sodium hydrogensulfite, sodium thioglycolate, potassium pyrosulfite, sodium pyrosulfite, α-thioglycerin, ethylene glycol, dimethylsulfoxide, sodium sulfite, and sodium erythorbate.

3. The camptothecin compound-containing aqueous pharmaceutical preparation according to claim 1 or 2, wherein the phosphoric acid salt is an alkali metal phosphate.

4. The camptothecin compound-containing aqueous pharmaceutical preparation according to claim 1 or 2, wherein the phosphoric acid salt is sodium phosphate or potassium phosphate.

5. The camptothecin compound-containing aqueous pharmaceutical preparation according to any one of claims 1 to 4, which has a pH of 2 to 5.

6. The camptothecin compound-containing aqueous pharmaceutical preparation according to any one of claims 1 to 5, wherein the camptothecin compound is 7-ethyl-10-piperidinopiperidinocarbonyloxycamptothecin.

7. The camptothecin compound-containing aqueous pharmaceutical preparation according to any one of claims 1 to 6, which is an anti-tumor pharmaceutical preparation.

8. The camptothecin compound-containing aqueous pharmaceutical preparation according to any one of claims 1 to 7, which is an injection pharmaceutical preparation.
